(19) Europäisches Patentamt / European Patent Office / Office européen des brevets

(11) **EP 4 398 132 A1**

(12) **EUROPEAN PATENT APPLICATION**

(43) Date of publication:
**10.07.2024 Bulletin 2024/28**

(21) Application number: **23382001.8**

(22) Date of filing: **03.01.2023**

(51) International Patent Classification (IPC):
**G06F 18/2413** (2023.01)    **A61B 5/00** (2006.01)
**G06T 7/00** (2017.01)    **G16H 50/20** (2018.01)

(52) Cooperative Patent Classification (CPC):
**G06F 18/24133; G06T 7/0012; G16H 30/40;
G16H 50/20;** G06V 10/82; G06V 2201/031

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Centre De Visió Per Computador
08193 Cerdanyola del Vallès (ES)**
• **Fundació Institut d'Investigació en
Ciències de la Salut Germans Trias i Pujol (IGTP)
08916 Badalona (ES)**
• **Universitat Autónoma de Barcelona
08193 Bellaterra (ES)**

(72) Inventors:
• **GIL RESINA, Debora
08005 Barcelona (ES)**
• **SANCHEZ RAMOS, Carles
08006 Barcelona (ES)**
• **ROSELL GRATACÓS, Antoni
08037 Barcelona (ES)**
• **BAEZA MENA, Sonia Margarita
08032 Barcelona (ES)**
• **TORRES, Guillermo Eduardo
08030 Barcelona (ES)**

(74) Representative: **Torner, Juncosa I Associats, SL
C / Pau Claris, 108, 1r 1a
08009 Barcelona (ES)**

(54) **A COMPUTER-IMPLEMENTED METHOD, A SYSTEM AND COMPUTER PROGRAMS FOR CHARACTERIZATION OF PATHOLOGIES**

(57)    A method, system and computer programs for characterization of pathologies are provided. The method comprises accessing or receiving image data and clinical and demographic data and/or genomic information, the image data including different types of scans of a subject, and the clinical and demographic data including attributes of a group of subjects; computing radiomic features using a feature extraction algorithm and embedding the computed radiomic features and the clinical and demographic data and/or genomic information in an abstract space representing the lesion; performing a characterization of the lesion at different levels by determining categorical and quantitative variables associated to each level using the computed radiomic features, providing a multi-diagnostic and/or -prognostic characterization of the lesion as a result; and transforming the provided multi-diagnostic and/or -prognostic characterization of the lesion into radiological words or annotations that quantitatively and/or qualitatively describe the lesion using deep learning or machine learning algorithms.

Fig. 1

**Description**

Technical Field

The present invention relates to a computer implemented method, to a system, and to computer programs for the characterization of pathologies, including lung pathologies, cardiac pathologies, brain pathologies, cancers, inflammations, infections, etc. of any organ or body tissue of a subject.

The aim of the present invention is to integrate radiological images, and at least one of clinical and demographic data or genomic information, to achieve an explainable digital support diagnosis to maximize efficient decision making and improve the emotional burden on a subject.

Background of the Invention

With regard to pathology diagnosis, the application of radiomics [1] (a recent discipline that extracts a large number of image features correlating to treatment outcome), could represent a critical shift in the reduction of the false positive rate and an improvement of early diagnosis of lung cancer. In this sense, in the retrospective pilot study reported in Hawkins et al [2] a random forest classifier of 150 quantitative image features obtained a significantly better predictive value than volumetry alone (AUC= 0.87 vs 0.74). Peikert et al. [3] built a radiomic classifier based upon 8 quantitative radiologic features capturing location, size, shape descriptors and texture analysis that achieved an AUC of 0.939 with 90% of sensitivity for malignancy detection. An alternative to classic radiomic approaches is machine learning, which uses well known descriptors (like Gabor, Local Binary Patterns -LBP-, or SIFT) to represent a nodule in an embedding feature space, which is the input to a classifier. These methods achieve better diagnostic power than radiomic methods with AUC equal to 0.97, sensitivity equal to 96% with 95% of specificity [4].

Inspired by the great success in other areas of application, in recent times several deep Convolutional Neural Network (CNNs) have been used to classify malign and benign nodules. A main challenge especially for training new models with complex architectures [5-9], is the limited amount of good quality data with annotations. Current approaches are based on either 3D analysis of volumes or, in an attempt to augment the available data, an aggregation of 2D analysis at slice level. 3D approaches are hindered by the limited amount of annotated data [5-6], while 2D analysis must manage the embedding of a variable number of 2D slices into a common feature space to allow for a diagnosis based on the whole volume [7,8] . In any case, their accuracy is at most 90% with an unbalance between sensitivity and specificity which does not outperform conventional machine learning approaches like the one in [4]. Another pitfall, especially for deep methods, is that models should be easily interpreted from a clinical point of view to allow the analysis of the clinical factors that have an impact on the clinical decision [9].

18F-fluorodeoxyglucose (FDG) positron emission tomography is a non-invasive imaging technique that reflects cell metabolism and is mainly indicated in diagnosing lymph node and distant metastases, assessing treatment response, and detecting recurrence after treatment in various malignancies. Generally, tumour FDG uptake shows uneven spatial distribution due to underlying biological tumour conditions such as metabolism, hypoxia, necrosis, and cellular proliferation, which is referred to as intratumoral heterogeneity. However, although intratumoral heterogeneity is related to tumour aggressiveness, treatment response, and prognosis, established FDG PET parameters such as maximum standardized uptake value (SUV), metabolic tumour volume (MTV), and total lesion glycolysis (TLG) do not reflect this property, raising the need for different analytic methods. Texture analysis reflects tumour heterogeneity and has been recently introduced in the analysis of PET images. It has demonstrated its value in establishing survival [10], predicting distant metastasis [11], detecting mutations [12] and establishing radiotherapy doses [13]. However, there are some limitations like the low reliability of heterogeneity parameters in tumours with small volume, the low repeatability and reproducibility of textural features in the clinical setting and the limitation of the analytic methods. This could be corrected by the introduction of a better description of the intrinsic heterogeneous structure of tumours [14] and the fusion of PET-CT data at structural (representing tumour heterogeneity) and content (representing tumour composition, density in CT and metabolism in PET).

Radiogenomics is a field of radiomics dedicated to identifying genomic alterations within tumor DNA, which is essential for targeted therapy and for predicting response to both chemotherapy and radiotherapy. Aerts et al. were able to estimate epidermal growth factor receptor (EGFR) mutation status based on a mixture of volume, texture, and gradient features from data extracted from pre-treatment computed tomography (CT) scans [15]. A more recent study [16] demonstrated radiogenomic associations between CT features and the EGFR mutation, ALK rearrangement and the KRAS mutation [16]. To date, only common morphometabolic radiological features have been described with EFGR, ALK, KRAS, RET and ROS1, PD-L1.

To the inventor's knowledge, there is no software for clinical use that applies radiomics and radiogenomics to the early diagnosis of lung cancer or even of other respiratory diseases in a screening program. The integration of imaging, genomics, metabolomics, and clinical data to decode different types of tissue biology in LC screening have great potential to improve screening profitability and make lung cancer screening programs cost-effective so that they can be implemented in a sustainable health-care system [17].

With regard to explainability, many works addressing radiological interpretation with deep networks rely on the identification of the image regions that the network pays attention to. This identification is computed by back-propagating the content of intermediate layers and using attention models [18]. Attention models have gained increasing interest to improve segmentation, detection, and final diagnosis classification of lesions [19, 20]. From these works, it has been demonstrated that the use of self-attention in a transformer architecture accurately improves results [21].

Given that attention models dynamically compute a weight vector to extract partial image features supporting the final prediction, the visualization of the associated attention maps allows the identification of regions relevant to the model's output. Visualization of attention maps provides a level of transparency that allows users to validate model outputs, but still lacks the link to the clinical domain knowledge. A recent study [22] shows that to facilitate the use of AI models in clinical practice, clinicians need to justify their decision-making based on the model's prediction in terms of the current evidence based medical practice. Indeed, the combination of radiological and visual deep features related to malignancy has shown to improve interpretability of results [23]. Therefore, some recent works addressing automatic report generation have been published. In [24, 25], the authors present models that combine attention maps and radiological reports to predict lesion pathology and automatically generate reports for new cases. Also, preliminary studies with simple forms of self-attention have shown its usefulness in medical imaging segmentation, classification, detection, reconstruction, registration, synthesis, and clinical report generation [26].

Finally, in [27] several models directly linking abstract features to radiological descriptions are presented and tested for the characterization and diagnosis of lesions. All these works are end-to-end approaches that use a combination of medical images and radiological annotations. Thus, they are unable to provide an interpretation of pre-trained visual features.

Current deep approaches are black-box models that provide clinical outcomes directly from scans and, thus, their output is even harder to interpret than other less accurate systems based on hand-crafted features explicitly describing the morphology and shape of lesions. Although activation and attention maps of the neurons improve interpretability and trustworthiness of deep approaches, they do not have a straightforward correspondence with the annotations that a radiologist would made to summarize the clinical findings of the lesion.

Besides the above, nowadays various softwares or solutions for clinical use are currently available in the market. These softwares or solutions can automatically detect and segment body nodules, calculate its volume and its doubling time, either as independent software from the radiology equipment or integrated within of the Computed Tomography (CT) equipment. However, the known solutions do not provide precise information on the histology and molecular diagnosis of the PNs, neither their prognosis.

Finally, with regard to hyperparameter optimization, Deep Learning has enabled remarkable progress over the last years on a several objectives, such as image and speech recognition, and machine translation. Deep neural architectures are a main contribution for this progress. Current architectures have mostly been developed manually by engineers, which is a time-consuming and error-prone process. Because of this, there is growing interest in automated neural architecture search methods.

Meta-learning, or learning to learn, is the science of systematically observing how different machine learning approaches perform on a given learning task, and then learning from this experience, or meta-data, to learn which approach is the optimal one for the task [28]. This not only accelerates and improves the design of machine learning or neural architectures pipelines, but it also allows to replace hand-designed algorithms with new learning data-based approaches. One of the most important meta-learning sub fields is automated hyper-parameter optimization (HPO). HPO finds a tuple of hyper-parameters that yields an optimal model which minimizes a predefined loss function on given independent data [29]. HPO has several outcomes: 1) reduce the human effort applying at hand machine learning configurations; 2) improve the performance of machine learning algorithms; and 3) improve the reproducibility and fairness of scientific studies.

In particular, if only a single approach is considered, HPO techniques can be applied to optimize its hyperparameters. In the case of a neural network, the set of hyper-parameters define its architecture, as well as, its training, which includes backpropagation configuration (like learning rate) and the definition of the loss function weights. Any strategy for HPO should include three main steps: search space, search strategy, and performance estimation.

First, the parameter-search-space (statically constructed by the user) is defined by a tuple of hyper-parameters and its possible discrete value ranges where, the space dimensionality is the number of hyper-parameters, and its search area is bounded by the value ranges. Each tuple of hyper-parameters values define a candidate network to be the optimal one [30-31]. Many approaches have been proposed in order to select a candidate network or apply a search strategy.

Second, search strategies can be grouped into two baseline methodologies: exhaustive and evolutionary searching. In one hand, exhaustive or brute-force search (like grid search or random search [32]) consists of systematically enumerating all possible candidates for the solution and checking whether each candidate satisfies the objective. On the other hand, evolutionary search (like Bayesian [33] or evolutionary genetic [34] algorithms) chooses the new candidates based on the knowledge achieved by previous ones. In [35], the authors report a comparison between these strategies.

Third, regardless of the search strategy used, a critical point is how to define the metrics evaluating the performance (i.e. the radiomic features) and generalization power of the different candidate networks. The simplest approach is to apply a single-objective function over a tuple of hyper-parameters and returns the associated loss [29]. Then k-fold or cross-validation applied to the training set [36] or evaluation to a hold out [37] are often used to estimate this generalization performance [5]. Nowadays the usual approach is to apply a nested cross-validation procedure where hyperparameter selection is performed in the inner cross-validation, while the outer cross-validation computes an unbiased estimate of the expected accuracy of the algorithm [38].

**References**

[1] Xie, H.; Yang, D.; Sun, N.; Chen, Z.; Zhang, Y. Automated pulmonary nodule detection in CT images using deep convolutional neural networks. Pattern Recognition 2019, 85, 109-119.

[2] Hawkins et al. Prediction of pathological nodal involvement by CT-based Radiomic features of the primary tumor in patients with clinically node-negative peripheral lung adenocarcinomas, Med. Phys. 45 (6), 2018.

[3] Peikert T et al. Novel high-resolution computed tomography-based radiomic classifier for screen-identified pulmonary nodules in the National Lung Screening Trial, PLOS ONE 13(10), 2018.

[4] Zhang, F.; et al. Lung nodule classification with multilevel patch-based context analysis. IEEE Biom Eng., 2013, 61, 1155-1166.

[5] Jiang, H.; Shen, F.; Gao, F.; Han,W. Learning efficient, explainable and discriminative representations for pulmonary nodules classification. Pattern Recognition 2021, 113, 107825.

[6] Zhu, W.; et al. Deeplung: Deep 3d dual path nets for automated pulmonary nodule detection and classification. WACV, 2018, pp. 673-681.

[7] Yan, X.; et al. Classification of lung nodule malignancy risk on computed tomography images using convolutional neural network: A comparison between 2d and 3d strategies. ACCV., 2016, pp. 91-101.

[8] Shen, W.; et al, J. Multi-crop convolutional neural networks for lung nodule malignancy suspiciousness classification. Pattern Recognition 2017, 61, 663-673.

[9] Maicas, G.; at al. Training medical image analysis systems like radiologists. MICCAI, 2018, pp. 546-554.

[10] Ohri N, Duan F, Snyder BS, Wei B, Machtay M, Alavi A, et al. Pretreatment 18F-FDG PET textural features in locally Advanced non-small cell lung cancer: secondary analysis of ACRIN 6668/RTOG 0235. J Nucl Med.57:842-8, 2016.

[11] Wu J,Aguilera, et al. Early-stage non-small cell lung cancer: quantitative imaging characteristics of 18F-fluorodeoxyglucose PET/CT allow prediction of distant metastasis. Radiology, 281 :270-8, 2016.

[12] Yip SS, et al. Associations between somatic mutations and metabolic imaging phenotypes in non-small cell lung cancer. J Nucl Med. 58:569-76, 2017.

[13] Fried DV, Mawlawi O, Zhang L, Fave X, Zhou S, Ibbott G, et al. Potential use of (18)F-fluorodeoxyglucose positron emission tomography-based quantitative imaging features for guiding dose escalation in stage III non-small cell lung cancer. Int J Radiat Oncol Biol Phys. 2016;94:368-76.

[14] Bernatowicz K et al, Robust imaging habitat computation using voxel-wise radiomics features, SciReports, 2021.

[15] Aerts HJ, et al. Defining a Radiomic Response Phenotype: A Pilot Study using targeted therapy in NSCLC. Sci Rep. 2016; 6:33860.

[16] Rizzo S et al. Genomics of non-small cell lung cancer (NSCLC): Association between CT based Imaging features and EGFR and K-RAS mutations in 122 patients-An external validation. Eur J Radiol. 2019;110:148-155.

[17] Goulart B, et al, Lung cancer screening with low-dose computed tomography: costs, national expenditures, and cost-effectiveness, J Natl Compr Canc Netw, 2012 Feb;10(2):267-75.

[18] Vasilakakis, M., Sovatzidi, G., Iakovidis, D.K.: Explainable classification of weakly annotated wireless capsule endoscopy images based on a fuzzy bag-of-colour features model and brain storm optimization. In: International Conference on Medical Image Computing and Computer-Assisted Intervention. pp. 488-498. Springer (2021).

[19] Gao, Y., Zhou, M., Metaxas, D.N.: Utnet: a hybrid transformer architecture for medical image segmentation. In: International Conference on Medical Image Computing and Computer-Assisted Intervention. pp. 61-71. Springer (2021).

[20] Jiang, H., Shen, F., Gao, F., Han, W.: Learning efficient, explainable and discriminative representations for pulmonary nodules classification. Pattern Recognition 113, 107825 (2021).

[21] Shamshad, F., Khan, S., Zamir, S.W., Khan, M.H., Hayat, M., Khan, F.S., Fu, H.: Transformers in medical imaging: A survey. arXiv preprint arXiv:2201.09873 (2022).

[22] Tonekaboni, S., Joshi, S., McCradden, M.D., Goldenberg, A.: What clinicians want: contextualizing explainable machine learning for clinical end use. In: Machine learning for healthcare conference. pp. 359-380. PMLR (2019).

[23] Zheng, S., Shen, Z., Pei, C., Ding, W., Lin, H., Zheng, J., Pan, L., Zheng, B., Huang, L.: Interpretative computer-aided lung cancer diagnosis: From radiology analysis to malignancy evaluation. Computer Methods and Programs in Biomedicine 210, 106363 (2021).

[24] Lee, H., Kim, S.T., Ro, Y.M.: Generation of multimodal justification using visual word constraint model for explainable computer-aided diagnosis. In: Interpretability of Machine Intelligence in Medical Image Computing and Multimodal Learning for Clinical Decision Support, pp. 21-29. Springer (2019).

[25] Wang, X., Peng, Y., Lu, L., Lu, Z., Summers, R.M.: Tienet: Text-image embedding network for common thorax disease classification and reporting in chest x-rays. In: Proceedings of the IEEE conference on computer vision and pattern recognition, pp. 9049-9058 (2018)

[26] Shamshad, F., Khan, S., Zamir, S.W., Khan, M.H., Hayat, M., Khan, F.S., Fu, H.: Transformers in medical imaging: A survey. arXiv preprint arXiv:2201.09873 (2022).

[27] Marques, S., Schiavo, F., Ferreira, C.A., Pedrosa, J., Cunha, A., Campilho, A.: A multi-task CNN approach for lung nodule malignancy classification and characterization. Expert Systems with Applications 184, 115469 (2021).

[28] J. Waring, C. Lindvall, and R. Umeton, "Automated machine learning: Review of the state-of-the-art and opportunities for healthcare," Artificial intelligence in medicine, vol. 104, p. 101822, 2020.

[29] M. Claesen and B. De Moor, "Hyperparameter search in machine learning," arXiv preprint arXiv:1502.02127, 2015.

[30] T. Akiba, S. Sano, T. Yanase et al., "Optuna: A next-generation hyperparameter optimization framework," in Proceedings of the International Conference on Knowledge Discovery and Data Mining, 2019.

[31] R. Andonie, "Hyperparameter optimization in learning systems," Journal of Membrane Computing, vol. 1, no. 4, pp. 279-291, 2019.

[32] J. Bergstra and Y. Bengio, "Random search for hyper-parameter optimization." Journal of machine learning research, vol. 13, no. 2, 2012.

[33] J. Wu, X.-Y. Chen, H. Zhang, L.-D. Xiong, H. Lei, and S.-H. Deng, "Hyperparameter optimization for machine learning models based on bayesian optimization," Journal of Electronic Science and Technology, vol. 17, no. 1, pp. 26-40, 2019.

[34] N. M. Aszemi and P. Dominic, "Hyperparameter optimization in convolutional neural network using genetic algorithms," International Journal of Advanced Computer Science and Applications, vol. 10, no. 6, 2019.

[35] H. Alibrahim and S. A. Ludwig, "Hyperparameter optimization: comparing genetic algorithm against grid search and bayesian optimization," in 2021 IEEE Congress on Evolutionary Computation (CEC). IEEE, 2021, pp. 1551-1559.

[36] C.-W. Hsu, C.-C. Chang, C.-J. Lin et al., "A practical guide to support vector classification," 2003.

[37] D. Chicco, "Ten quick tips for machine learning in computational biology," BioData mining, vol. 10, no. 1, pp. 1-17, 2017.

[38] J. Wainer and G. Cawley, "Nested cross-validation when selecting classifiers is overzealous for most practical applications," Expert Systems with Applications, vol. 182, p. 115222, 2021.

Description of the Invention

The present invention provides a one-shot multi-level diagnosis support solution that uses a combined radiomic analysis approach with three sublevels (clinical, histopathological, and molecular) to reduce false positives in diagnosis. The invention can use a hybrid system that analyzes embedded radiomic features to characterize nodules and an optimized feed forward structure to identify nodules, thereby producing a reliable method of malignancy detection. The invention will interpret the radiomic features of the images/scans into understandable radiological annotations to increase clinical utility. The invention can analyze the subject different medical image scans, related genomic information and/or clinical and demographic data, fully inside a standalone server, thus acting as a software-as-a-service model.

The most innovative aspects of the present invention can be summarized as follows:

1. Multi-level diagnosis support module: permits malignancy predictions with high specificity and sensitivity, which decreases the rate of false positives.

2. Explainability module: increases the clinical relevance of data and allows physicians to make better treatment decisions.

3. Module Hyperparameter optimization.

4. Diagnosis support-as-a-service with self-contained server allows the software to be run remotely and makes it more accessible.

Particularly, the proposed invention is based on radiomics and radio-genomics for the characterization of pathologies. It is mainly based on a software solution for the early characterization of pathologies, which will avoid unnecessary procedures and surgeries, improve the quality of life of the participants and optimize the necessary resources at the health care level.

The invention develops and clinically tests an artificial intelligence system based on CNNs for the early characterization of pathologies by extracting and analyzing multimodal radiomic data. After detecting a lesion in a CBCT or other imaging technology, it will be possible to predict the probability of malignancy of the lesion and its mutational profile and evolution.

In addition to image analysis of pathologies using artificial intelligence and convolutional neural networks applied to radiomics, clinical and demographic data and/or genetic information will be correlated to obtain a personalized diagnosis support and identify different patient profiles.

The present invention will contribute to and fill two main gaps the provision and improvement of the performance of a new method by accessing and testing new data, and the easy and fast access of the latter to the clinical community.

An object of present invention is thus to provide a new characterization method and system for pathologies, of any kind, such as cancers, lung/cardiac/brain/skin pathologies, among many others.

This object is fulfilled by a method with the characteristics of claim 1, by a system with the features of claim 11 and by a computer program with the features of claim 15.

To that end the present invention proposes, according to one aspect, a computer-implemented method for characterization of pathologies. The method comprises: accessing or receiving, by a processor, stored image data and clinical and demographic data and/or genomic information, the image data including different types of 2D and/or 3D scans from a body area or tissue of a subject, and the clinical and demographic data including attributes of at least one group of patients/subjects; computing, by a processor, for a given defined region of interest containing a lesion to be analyzed in the 2D or 3D scans, radiomic features using a feature extraction algorithm applied to the input scans, and embedding the computed radiomic features and the clinical and demographic data and/or genomic information in an abstract space representing the lesion; performing, by a processor, a characterization of the lesion at different levels (of detail) by means of determining categorical (discrete) and/or quantitative (continuous) variables associated to each level of said different levels using the abstract space representing the lesion, providing a multi-diagnostic and/or -prognostic characterization of the lesion as a result, the different levels including two or more of the following: a clinical diagnosis support indicating malignancy or benignancy of the lesion; a histological diagnosis support indicating a typology of the lesion; a genomic diagnosis support indicating a (possible) best treatment; an epigenomic diagnosis support indicating which chromosomes are associated with the lesion; and transforming, by a processor, using one or more deep learning or machine learning algorithms, the provided multi-diagnostic and/or -prognostic characterization of the lesion into radiological words or annotations that quantitatively and/or qualitatively describe the lesion.

Present invention also proposes, according to another aspect, a system for characterization of pathologies, comprising: a memory or database configured to store image data, genomic information and clinical and demographic data, the image data including different types of 2D and/or 3D scans from a body area or tissue of a subject, and the clinical and demographic data including attributes of at least one group of patients/subjects; and one or more processors configured to perform the following tasks: access or receive the stored image data and clinical and demographic data and/or genomic information; compute, for a given defined region of interest containing a lesion to be analyzed in the 2D and/or 3D scans radiomic features using a feature extraction algorithm applied to the input scans, and embedding the computed radiomic features and the clinical and demographic data and/or genomic information in an abstract space representing the lesion; perform a characterization of the lesion at different levels (of detail) by means of determining categorical (discrete) and/or quantitative (continuous) associated to each level of said different levels using the abstract space representing the lesion, providing a multi-diagnostic and/or -prognostic characterization of the lesion as a result, the different levels including two or more of the following: a clinical diagnosis support indicating malignancy or benignancy of the lesion; a histological diagnosis support indicating a typology of the lesion; a genomic diagnosis support indicating a (possible) best treatment; an epigenomic diagnosis support indicating which chromosomes are associated with the lesion; and transform, using one or more deep learning or machine learning algorithms, the provided multi-diagnostic and/or -prognostic characterization of the lesion into radiological words or annotations that quantitatively and/or qualitatively describe the lesion.

The system can comprise a plurality of processors where each processor can be configured to perform one or more of said tasks and can be implemented in a computing module of a plurality of computing modules operatively connected via a communications network implementing a cloud-based distributed system architecture.

Likewise, other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

It should be noted that in some embodiments the 2D/3D scans can be processed in real time.

According to the invention, the feature extraction algorithm can comprise hand-crafted classic machine learning feature extractors, including a Local binary patterns (LBP) algorithm; a PyRadiomics algorithm; and/or a Histogram of Oriented Gradients (HoG) algorithm, as well as, deep features obtained from a pre-trained convolutional neural network with/without fine tuning or an own designed specific deep learning algorithm trained from scratch, or even one or more neural networks transforming any of the above features into a common radiomic space combining one or more of them.

PyRadiomics features can include shape features of the lesion, first order features, and/or textural features (Gray Level Co-ocurrence Matrix (GLCM), Gray Level Size Zone (GLSZM), Gray Level Run Length Matrix (GLRLM) and Gray Level Dependency Matrix (GLDM)) describing several aspects of the lesion. The features can be selected according to either the reproducibility against different image acquisition conditions and interobserver variability in lesion identification or correlation to malignancy.

In some embodiments, the radiomic features comprise one or more of: texture features, shape features, size features, asymmetry features, density features, features quantifying homogeneity and/or heterogeneity of the lesion, calcification, texture, sphericity, among others.

According to the invention, the 2D and/or 3D scans can be acquired using any of the following technologies: Computed Tomography (CT); Perfusion Single Photon Emission Computed Tomography (Q-SPECT); Photon Emission Tomography (PET); Magnetic Resonance Imaging (MRI); X-rays; echography, etc.

The attributes included in the clinical and demographic data can comprise the age or average age of the subjects, the gender of the subjects, an indication of whether the subjects have suffered an infectious disease, type of diet followed by the subjects, weight, fat level, family history of previous illnesses, pollution levels, smoking habits, sport habits, among many others.

In some embodiments, the categorical variables can be determined/predicted by minimizing a multi-label classification loss function and the quantitative variables can be determined/predicted by minimizing a regression function.

In some embodiments, a loss function of each determined categorical and/or quantitative variable is also optimized. This can be done using different strategies/approaches, for example, by using two different systems, one for the categorical variables and one for the quantitative variables; by using one single system that optimizes a single loss function combining the different loss functions used to predict each type of variable (categorical, quantitative); or even by using one single system that optimizes each loss function independently using a multi-objective approach.

In some embodiments, the one or more deep learning or machine learning algorithms used in the transforming step comprises a multi-attention transformer.

In some embodiments, hyperparameters of the one or more deep learning or machine learning algorithms are optimized by using a gradient-descent (backpropagation) of the loss function and/or a multi-objective evolutionary computation scheme based, for instance, on Pareto front selection.

Hyperparameters define the different architectures of a given network model and, thus, they parameterize the space of networks. Although some of them might be specific to the model, usually they include the number of layers, neurons for each layer and kernel size (in case of convolutional models). Aside network architecture, there are some hyperparameters related to the backpropagation training that also have an impact on performance and, thus, they could be also optimized. These parameters include the learning rate (lr) of the backpropagation and the dropout rate of the network. Finally, for some multi-task problems, the loss function is given by a weighted average of each specific task loss. These weights should be also optimized in order to obtain good performance. Therefore, the search space, $\Theta$, that in some embodiments is proposed for network meta-learning is given by: $\Theta$ = (Architecture Parameters; Training Parameters; and/or Loss Parameters).

In some embodiments, the accessing/receiving step further comprises accessing sensor signals associated to the subject, the sensor signals being also used in the computing step of the radiomic features.

In a particular embodiment, the presents invention formulates interpretability of AI models as a transformation between the domains of radiomic visual features and radiological descriptions. Transformers are particularly used to map abstract radiomic features to specific radiological annotations. Moreover, according to the present invention, in the radiomic domain, lesions are described as a sentence given by a self-defined embedding of their visual features. This sequence of visual features is the input to a multi-attention transformer optimizing a hybrid loss function that translates the visual sequence into a sequence of radiological words describing the lesion appearance in quantitative and/or qualitative terms. The network hyperparameters of the transformer could be optimized, for instance, using a Non-dominated Sorting Genetic Algorithm (NSGA-II) or another multi-objective optimization search strategy.

Therefore, present invention provides a new solution based on a multi-radiomic signature to support decision-making for clinicians that can accurately discriminate between cancer and benign nodules, or between unhealthy and healthy tissues or organs. This approach improves disease screening programs efficacy and efficiency, reducing patient waiting lists and the harm and psychological burden on patients, and improving patient safety and quality of life.

The proposed radiomics solution for the multi-diagnosis support of pathologies helps to reduce the workload of doctors, facilitate hospital management and reduce the costs associated with carrying out multiple tests. Moreover, radiology also improves the quality of life of the patient, since the diagnosis support is made through images, i.e. a non-invasive clinical procedure.

The present invention can be defined as a software tool for daily clinical use in a cancer screening program, being in some of the embodiments implemented as a cloud service, a SaaS client-server architecture for diagnosis support as a service (DaaS); as it does not require the set-up of applications on the clients' own computers, allowing its use without additional hardware or licensing costs.

Brief Description of the Drawings

The previous and other advantages and features will be more fully understood from the following detailed description of embodiments, with reference to the attached figures, which must be considered in an illustrative and non-limiting manner, in which:

Fig. 1 is a flowchart illustrating a method for characterization of pathologies, according to an embodiment of the present invention.

Fig. 2 illustrates the transformer used for model interpretability, according to an embodiment of the present invention.

Fig. 3 illustrates the meta-learning step approach based on multiobjective optimization integrated in a nested Cross-Validation schemed, according to an embodiment of the present invention.

Fig. 4 graphically illustrates the proposed DaaS architecture, according to an embodiment of the present invention.

Detailed Description of the Invention and of Preferred Embodiments

Present invention provides an integrative method and system based on intelligent radiomic analysis of multimodal radiological and clinical data investigating lesions at multilevel diagnostic and/or prognostic. The method and system are based on a multimodal approach that embeds lesions into an abstract radiomic space defined by machine learning and/or deep radiomic features describing the lesion morphology and metabolic activity, clinical, demographic and genomic data. These features are the input to a fully connected network with an architecture optimized to ensure maximum clinical outcome. The intelligent radiomic system has three main steps. First, 2D-3D scans are pre-processed. Second, radiomic features are extracted from the registered volumes of interest in the 2D-3D scans to define a radiomic feature space and embedded with clinical and demographic data and/or genomic information in an abstract space representing the lesion. Finally, a machine learning method is used to disseminate each value of the feature space between the different types of lesion patterns.

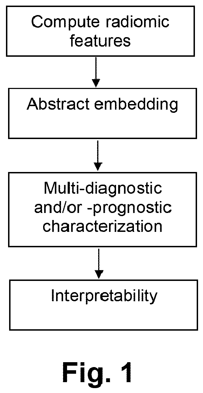

Fig. 1 illustrates the main steps executed by the proposed method to characterize a pathology. The method can take or receive as input data: 2D/3D scans (e.g. CT, PET, MRI, among other types of images), clinical and demographic data (e.g. age or average age, gender, infectious disease or cancer antecedents, diet, family history of previous illnesses, smoking habits, level of pollution of the place where the subjects live, etc.), genomic information, and optionally sensor signals, and for each region of interest (2D and/or 3D) containing a lesion to be analyzed/characterized it computes radiomic features and embeds the computed radiomic features, the clinical and demographic data and/or the genomic information, and also, optionally, the sensor signals, in an abstract space representing the lesion using either classic (like PyRadiomics, texture features, LBP, HoG) or deep (extracted using a convolutional, fully connected network, transformers or recurrent networks) approaches for feature extraction.

Self-attention mechanisms can be used to find nonlinear correlations between the different types of input data (images, clinic and demographic data, genomic information and/or sensor signals) and abstract embedding.

The multi-diagnostic and/or -prognostic characterization of the lesion is performed at different (diagnostic) levels. Thus, the abstract embeddings represented before are used as input for a fully connected network that predicts or determines several categorical and quantitative variables associated to each level. For example, the different levels particularly include: a clinical diagnosis support indicating malignancy or benignancy of the lesion; a histological diagnosis support indicating a typology of the lesion; a genomic diagnosis support indicating a (possible) best treatment; and/or an epigenomic diagnosis support indicating which chromosomes are associated with the lesion. In an embodiment, the categorical variables are predicted/determined using a multi-label classification loss function and the quantitative variables are posed as a regression problem.

In some embodiments, the architecture of the fully connected network can have a common part transforming the embedded abstract features followed by independent architectures for the separate optimization of each loss function.

Finally, the multi-diagnostic and/or -prognostic characterization of the lesion is transformed into radiological words or annotations that quantitatively and/or qualitatively describe the lesion using one or more deep learning and/or neural network and/or machine learning algorithms.

For the interpretability of the results (i.e. the transformation/translation of the output of the radiomic analysis into the radiological words or annotations) interpretability of AI/deep learning model is formulated as a transformation/translation between the domains of radiomic visual features and radiological descriptions. In a particular embodiment, the use of transformers to map the abstract radiomic features into the specific radiological annotations is proposed.

Moreover, in the radiomic domain, lesions can be described as a sentence given by a self-defined embedding of their visual features. This sequence of visual features can be the input to a multi-attention transformer optimizing a multi-objective loss function that transforms/translates the visual sequence into a sequence of radiological words describing the lesion appearance in quantitative and/or qualitative terms. This loss function can be further optimized either using a gradient-descent (backpropagation) of a hybrid loss defined as a weighted average of the multiple losses or a multi-objective evolutionary computation scheme based on Pareto front selection of a population of networks. In case of a hybrid loss, its weights can be optimized via a meta-learning step approach.

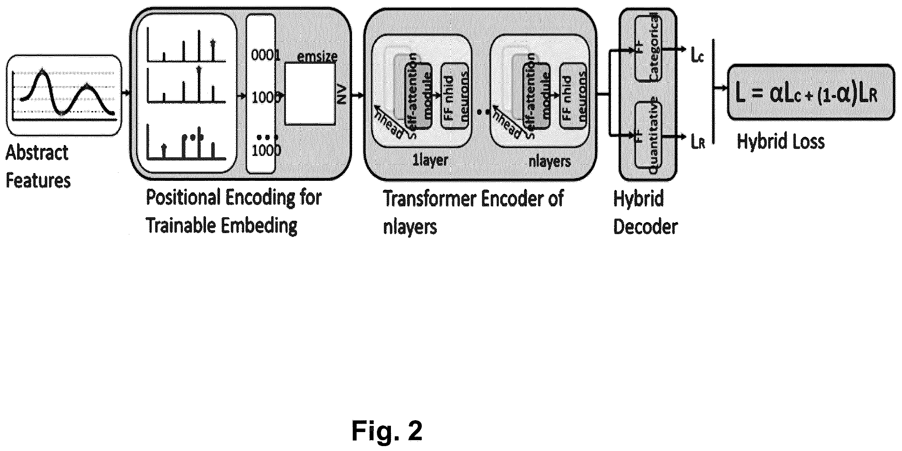

Fig. 2 illustrates an embodiment of the transformer. An encoder has a trainable embedding layer that maps a sequence of NV visual words to a Euclidean space of *emsize* dimensions. The embedded sequence words are the input to *nlayers* transformer identical layers. Each layer has two sub-layers. The first is a multi-head self-attention mechanism with *nhead,* and the second is a fully connected feed-forward layer with *nhid* neurons, Relu activation and dropout to mitigate over-fitting. The concatenation of the output of the last layer is the input to a decoder that computes two separate maps, one for each categorical and quantitative category of radiological attributes (i.e. computes the mapping between abstract and radiological features). The decoder is an ensemble of two fully connected layers one for each attribute type with sigmoid activation for the categorical decoder. The set of hyperparameters of the whole system are the network hyperparameters defining its architecture (*emsize, nhead, nhid, nlayers, dropout*), the weight $\alpha$ defining the hybrid loss and the learning rate (*lr*):

$$\Theta = (\text{Architecture Parameters; Training Parameters; and/or Loss Parameters}) = (\textit{emsize, nhead, nhid, nlayers, dropout, lr}).$$

Since the transformer is trained using a weighted binary cross entropy to account for cross annotations, in this case there are not hyperparameters associated to the definition of the loss. The input and output of a transformer are sequences of words represented as an index to an input and output corpus containing the collection of all input and output words. In present invention's case, the input is a NV -dimensional vector of radiomic visual features and, thus, they have to be coded as indexes in order to be the input for the transformer.

The input vector of visual features, $v = (v0, \ldots, v_{NV}$ -1) E [a0, b0] $\times \cdots \times$ [$a_{NV-1}$, $b_{NV-1}$] $\in$ $^{NV}$ is transformed to a sequence of NV visual words, $w^v = [w^v_0, \ldots, w^v_{NV-1}]$, using a discretization of each visual coordinate. For each coordinate, its range, [$a_j$, $b_j$], is discretized into nV uniform bins and $v_j$ is assigned to the index of the bin:

$$v_j \rightarrow \text{floor}((v_j - a_j)/(b_j - a_j) * (n_{V-1})) \in \{0, \ldots, nV - 1\}$$

for floor( · ) the integer part of a real number. The above transformation maps any coordinate to the same corpus of nV words indexed by {0, . . . , nV - 1}. In order to assign each coordinate to a different set of words, its index is shifted by j * nV positions to define $w^v_j$ :

$$w^v_j = \text{floor}((v_j - a_j)/(b_j - a_j) * (nV - 1)) + j * nV \in \{j * nV, \ldots, (j + 1) * nV - 1\}$$

This manner, v is mapped to a corpus of (NV - 1) * nV visual words indexed by {0, . . . , (NV -1)*nV -1}. It should be noted that the transformation assumes that the visual features are always in the same order, which is a reasonable assumption automatically fulfilled in the case of being the output of a network.

In a particular embodiment, the meta-learning step approach comprises, for all the modules/units of the proposed system (multi-diagnostic and/or -prognostic and interpretability), the implementation of a meta-learning of hyperparameters (architecture configuration and loss weights) using a multiobjective optimization scheme, like Non-dominated Sorting Genetic Algorithm (NSGA), integrated in a nested Cross-Validation (nCV) scheme for the computation of the radiomic features that define the objective functions. These objective functions are statistical summaries (average, $\mu$, and standard deviation, $\sigma$) of the losses obtained for a k-fold splitting of the training data. The units can be trained separately (in a two-step approach training first the multi-diagnostic and/or -prognostic module and using the trained module to train the interpretability of the results) or jointly using a common multi-objective strategy.

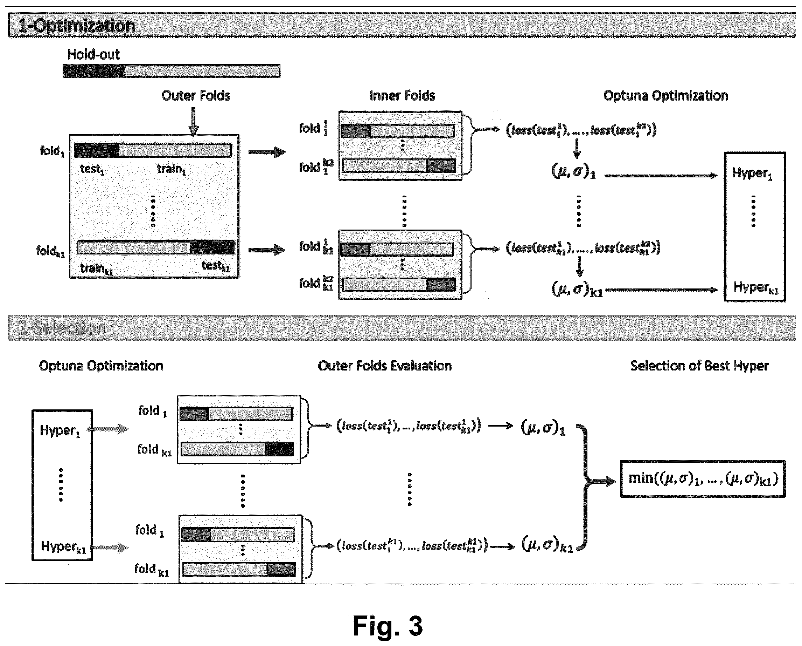

Fig. 3 illustrates an example of the proposed NSGA nCV scheme. The NSGA optimization process starts using the inner folds (top scheme) and with the final selection of the best hyperparameters using the outer folds (bottom scheme). First the input data is split into $k_1$-outer folds. For each outer fold, its train set is split into $k_2$-inner folds. The inner folds are used to optimize hyperparameters using NSGA framework, while the outer ones are used to select the best hyperparameters. To do so, for each outer fold, a hyperparameter configuration is trained and tested for all its inner folds using the network loss. The average and standard deviation of the loss function for the test sets of the $k_2$ inner folds are the objective functions to be used by a NSGA sampler in a multi-objective optimization strategy based on a dominated Pareto front selection. The NSGA optimization produces $k_1$ hyperparameter configurations (one for each outer-fold). These configurations are trained and tested in the $k_1$-outer folds and the one with the least average and standard deviation obtained for the test set is the one selected.

In some particular embodiments, the present invention is deployed in a flexible hybrid distributed modular architecture to be implemented in a client-server architecture for Diagnosis support as a Service (DaaS). The DaaS Architecture proposed particularly comprises a hybrid architecture. In this case, the invention's architecture or platform is implemented as a solution which offers the characteristics of distributed computing (cloud for offline processing and GPU/FPGA for real-time processing) to analyze, process, display, and planning bronchoscopies/biopsies, among others. Moreover, the invention will deliver the routes and guiding to possible body lesions as well as multidiagnostic screening within different levels. The architecture can support separate modules developed in different scripting languages. Those modules are designed using low coupling and high cohesion. The infrastructure enables the creation of alternative models for all different tasks.

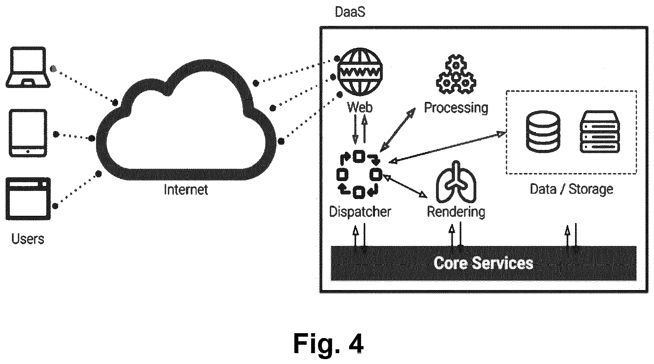

An embodiment of the proposed cloud-based architecture is described in Fig. 4, where modules are shown. The whole architecture allows the scalability and stability to update or incorporate modules. The backend modules are described in detail below:

- Web server: handle the requests clients connection in real-time, through a RESTful API
- Representational State Transfer.
- Dispatcher: administer all internal components in the DaaS architecture. This is the kernel for all operations: handling tasks, capturing exceptions, throwing I/O operations and others.
- Rendering: achieve the visualization and gather corresponding images from data to be send to the clients. The 3D rendering is achieved in server's memory when is requested employing a cache-based approach.
- Processing: process the tasks that could be launch asynchronously. In addition, this is capable of invoking scripts in other platforms and achieving the results for the application.
- Database: manage internal operations and underpin online interactions with clients under the Dispatcher control. Notice that physically database might be in another machine different than webserver.
- Storage: control the files that arrive or depart the backend (e.g. file request).
- Notice that I/O operations might cause error during file retrieval.
- Core Services: support for all modules offering a set of common backend functionalities. Internally, this module uses a class hierarchy for the scalability to support particular functions related with shared resources, neural network, synchronization, memory cache, and others.

Various aspects of the proposed method, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with image processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

A machine-readable medium may take many forms, including but not limited to, a tangible storage medium, a carrier wave medium or physical transmission medium. Non-volatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described herein may be embodied in a hardware device, it may also be implemented as a software only solution-e.g., an installation on an existing server. In addition, image processing as disclosed herein may be implemented as a firmware, firmware/software combination, firmware/hardware combination, or a hardware/firmware/software combination.

The scope of the present invention is defined in the following set of claims.

**Claims**

1. A computer-implemented method for characterization of pathologies, comprising:

accessing or receiving, by a processor, stored image data, clinical and demographic data and/or genomic information, the image data including different types of 2D and/or 3D scans from a body area or tissue of a subject, and the clinical and demographic data including attributes of at least one group of subjects;

computing, by a processor, for a given defined region of interest containing a lesion to be analyzed in the 2D and/or 3D scans, radiomic features using a feature extraction algorithm, and embedding the computed radiomic features and the clinical and demographic data and/or genomic information in an abstract space representing the lesion;

performing, by a processor, a characterization of the lesion at different levels by means of determining categorical and quantitative variables associated to each level of said different levels using the abstract space representing the lesion, providing a multi-diagnostic and/or -prognostic characterization of the lesion as a result, the different levels including two or more of the following: a clinical diagnosis support indicating malignancy or benignancy of the lesion; a histological diagnosis support indicating a typology of the lesion; a genomic diagnosis support indicating a best treatment; an epigenomic diagnosis support indicating which chromosomes are associated with the lesion; and

transforming, by a processor, using one or more deep learning or machine learning algorithms, the provided multi-diagnostic and/or -prognostic characterization of the lesion into radiological words or annotations that quantitatively and/or qualitatively describe the lesion.

2. The method of claim 1, wherein the feature extraction algorithm comprises:

a Local binary patterns, LBP, algorithm; a PyRadiomics algorithm; and/or a Histogram of Oriented Gradients, HoG, algorithm; or
a deep learning algorithm or one or more neural networks.

3. The method of any one of the previous claims, wherein the radiomic features comprise one or more of: texture features, shape features, size features, asymmetry features, density features, features quantifying homogeneity and/or heterogeneity of the lesion.

4. The method of any one of the previous claims, wherein the categorical variables are determined by means of minimizing a multi-label classification loss function and the quantitative variables are determined by means of minimizing a regression function.

5. The method of any one of the previous claims, further comprising optimizing a loss function of each determined categorical and/or quantitative variable by means of: two different systems, one for the categorical variables and one for the quantitative variables; one single system optimizing a single loss function combining different loss functions; or one single system optimizing each loss function independently using a multi-objective approach.

6. The method of any one of the previous claims, wherein the one or more deep learning or machine learning algorithms used in the transforming step comprises a multi-attention transformer.

7. The method of any one of the previous claims, further comprising optimizing several hyperparameters of the one or more deep learning or machine learning algorithms by either using a gradient-descent function or a multi-objective evolutionary computation scheme based on Pareto front selection.

8. The method of any one of the previous claims, wherein the 2D and/or 3D scans are acquired using two or more of the following technologies: Computed Tomography, CT; Perfusion Single Photon Emission Computed Tomography, Q-SPECT; Photon Emission Tomography, PET; Magnetic Resonance Imaging, MRI; X-rays; echography.

9. The method of any one of the previous claims, wherein the attributes included in the clinical and demographic data comprises at least one of the following: age or average age of the subjects, gender of the subjects, an indication of whether the subjects have suffered an infectious disease, type of diet, weight, family history of previous illnesses, smoking habits.

10. The method of any of the previous claims, wherein the accessing step further comprises accessing stored sensor signals associated to the subject, the sensor signals being also used in the computing step of the radiomic features.

11. A system for characterization of pathologies, comprising:

a memory or database configured to store image data, clinical and demographic data and/or genomic information, the image data including different types of 2D and/or 3D scans from a body area or tissue of a subject, and the clinical and demographic data including attributes of at least one group of subjects; and
one or more processors configured to perform the following tasks:

access or receive the stored image data and clinical and demographic data and/or genomic information;
compute, for a given defined region of interest containing a lesion to be analyzed in the 2D and/or 3D scans radiomic features using a feature extraction algorithm, and embedding the computed radiomic features and the clinical and demographic data and/or genomic information in an abstract space representing the lesion;
perform a characterization of the lesion at different levels by means of determining categorical and quantitative variables associated to each level of said different levels using the abstract space representing the lesion, providing a multi-diagnostic and/or -prognostic characterization of the lesion as a result, the different levels including two or more of the following: a clinical diagnosis support indicating malignancy or benignancy of the lesion; a histological diagnosis support indicating a typology of the lesion; a genomic diagnosis support indicating a best treatment; an epigenomic diagnosis support indicating which chromosomes are associated with the lesion; and
transform, using one or more deep learning or machine learning algorithms, the provided multi-diagnostic and/or -prognostic characterization of the lesion into radiological words or annotations that quantitatively and/or qualitatively describe the lesion.

12. The system of claim 11, comprising a plurality of processors, each processor of the plurality of processors being configured to perform one or more of said tasks and being implemented in a computing module of a plurality of computing modules, the plurality of computing modules being operatively connected via a communications network implementing a cloud-based distributed system architecture.

13. The system of claim 11 or 12, wherein the feature extraction algorithm comprises a Local binary patterns, LBP, algorithm; a PyRadiomics algorithm; a Histogram of Oriented Gradients, HoG, algorithm; a deep learning algorithm, and/or one or more neural networks, and wherein the one or more deep learning or machine learning algorithms used in the transforming step comprises a multi-attention transformer.

14. The system of claim 11, wherein the memory or database is further configured to store sensor signals associated to the subject, the one or more processors being further configured to use the stored sensor signals to compute the radiomic features.

15. A non-transitory computer readable medium including code instructions that when executed in a computer system implement the steps of:

receiving image data, clinical and demographic data and/or genomic information, the image data including different types of 2D and/or 3D scans from a body area or tissue of a subject, and the clinical and demographic data including attributes of at least one group of subjects;
computing, for a given defined region of interest containing a lesion to be analyzed in the 2D and/or 3D scans, radiomic features using a feature extraction algorithm, and embedding the computed radiomic features and the clinical and demographic data and/or genomic information in an abstract space representing the lesion;
performing, a characterization of the lesion at different levels by means of determining categorical and quantitative variables associated to each level of said different levels using the abstract space representing the lesion, providing a multi-diagnostic and/or -prognostic characterization of the lesion as a result, the different levels including two or more of the following: a clinical diagnosis support indicating malignancy or benignancy of the lesion; a histological diagnosis support indicating a typology of the lesion; a genomic diagnosis support indicating a best treatment; an epigenomic diagnosis support indicating which chromosomes are associated with the lesion; and
transforming, using one or more deep learning or machine learning algorithms, the provided multi-diagnostic and/or -prognostic characterization of the lesion into radiological words or annotations that quantitatively and/or qualitatively describe the lesion.

Compute radiomic features

↓

Abstract embedding

↓

Multi-diagnostic and/or -prognostic characterization

↓

Interpretability

# Fig. 1

Fig. 2

**Fig. 3**

**Fig. 4**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/211787 A1 (CHILDRENS HOSPITAL MED CT [US]) 21 October 2021 (2021-10-21) * paragraphs [0007] – [0009], [0012] – [0020], [0040] – [0045], [0049] – [0050], [0056], [0058], [0060], [0067], [0081], [0090], [0095], [0160] – [0162] * | 1-15 | INV. G06F18/2413 A61B5/00 G06T7/00 G16H50/20 |
| X | MICHELE AVANZO ET AL: "Machine and deep learning methods for radiomics", MEDICAL PHYSICS., vol. 47, no. 5, 1 May 2020 (2020-05-01), XP055719727, US ISSN: 0094-2405, DOI: 10.1002/mp.13678 * section 2 section 3 * | 1,11,15 | |

|  |
|---|
| TECHNICAL FIELDS SEARCHED (IPC) |
| G06V A61B G06F G06T G16H |

The present search report has been drawn up for all claims

| Place of search | Date of completion of the search | Examiner |
|---|---|---|
| The Hague | 16 May 2023 | Mukasa, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2001

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2023

| Patent document cited in search report | Publication date | Patent family member(s) | Publication date |
|---|---|---|---|
| WO 2021211787 A1 | 21-10-2021 | EP 4136658 A1 | 22-02-2023 |
| | | WO 2021211787 A1 | 21-10-2021 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XIE, H. ; YANG, D ; SUN, N. ; CHEN, Z. ; ZHANG, Y.** Automated pulmonary nodule detection in CT images using deep convolutional neural networks. *Pattern Recognition,* 2019, vol. 85, 109-119
- **HAWKINS et al.** Prediction of pathological nodal involvement by CT-based Radiomic features of the primary tumor in patients with clinically node-negative peripheral lung adenocarcinomas. *Med. Phys.,* 2018, vol. 45 (6
- **PEIKERT T et al.** Novel high-resolution computed tomography-based radiomic classifier for screen-identified pulmonary nodules in the National Lung Screening Trial. *PLOS ONE,* 2018, vol. 13 (10
- **ZHANG, F. et al.** Lung nodule classification with multilevel patch-based context analysis. *IEEE Biom Eng.,* 2013, vol. 61, 1155-1166
- **JIANG, H. ; SHEN, F. ; GAO, F. ; HAN,W.** Learning efficient, explainable and discriminative representations for pulmonary nodules classification. *Pattern Recognition,* 2021, vol. 113, 107825
- **ZHU, W. et al.** Deeplung: Deep 3d dual path nets for automated pulmonary nodule detection and classification. *WACV,* 2018, 673-681
- **YAN, X. et al.** Classification of lung nodule malignancy risk on computed tomography images using convolutional neural network: A comparison between 2d and 3d strategies. *ACCV.,* 2016, 91-101
- **SHEN, W. et al.** J. Multi-crop convolutional neural networks for lung nodule malignancy suspiciousness classification. *Pattern Recognition,* 2017, vol. 61, 663-673
- **MAICAS, G.** Training medical image analysis systems like radiologists. *MICCAI,* 2018, 546-554
- **OHRI N ; DUAN F ; SNYDER BS ; WEI B ; MACHTAY M ; ALAVI A et al.** Pretreatment 18F-FDG PET textural features in locally Advanced non-small cell lung cancer: secondary analysis of ACRIN 6668/RTOG 0235. *J Nucl Med,* 2016, vol. 57, 842-8
- **WU J,AGUILERA et al.** Early-stage non-small cell lung cancer: quantitative imaging characteristics of 18F-fluorodeoxyglucose PET/CT allow prediction of distant metastasis. *Radiology,* 2016, vol. 281, 270-8
- **YIP SS et al.** Associations between somatic mutations and metabolic imaging phenotypes in non-small cell lung cancer. *J Nucl Med.,* 2017, vol. 58, 569-76

- **FRIED DV ; MAWLAWI O ; ZHANG L ; FAVE X ; ZHOU S ; IBBOTT G et al.** Potential use of (18)F-fluorodeoxyglucose positron emission tomography-based quantitative imaging features for guiding dose escalation in stage III non-small cell lung cancer. *Int J Radiat Oncol Biol Phys.,* 2016, vol. 94, 368-76
- **BERNATOWICZ K et al.** Robust imaging habitat computation using voxel-wise radiomics features. *SciReports,* 2021
- **AERTS HJ et al.** Defining a Radiomic Response Phenotype: A Pilot Study using targeted therapy in NSCLC. *Sci Rep,* 2016, vol. 6, 33860
- **RIZZO S et al.** Genomics of non-small cell lung cancer (NSCLC): Association between CT based Imaging features and EGFR and K-RAS mutations in 122 patients-An external validation. *Eur J Radiol.,* 2019, vol. 110, 148-155
- **GOULART B et al.** Lung cancer screening with low-dose computed tomography: costs, national expenditures, and cost-effectiveness. *J Natl Compr Canc Netw,* February 2012, vol. 10 (2), 267-75
- Explainable classification of weakly annotated wireless capsule endoscopy images based on a fuzzy bag-of-colour features model and brain storm optimization. **VASILAKAKIS, M. ; SOVATZIDI, G. ; LAKOVIDIS, D.K.** International Conference on Medical Image Computing and Computer-Assisted Intervention. Springer, 2021, 488-498
- Utnet: a hybrid transformer architecture for medical image segmentation. **GAO, Y. ; ZHOU, M. ; METAXAS, D.N.** International Conference on Medical Image Computing and Computer-Assisted Intervention. Springer, 2021, 61-71
- **JIANG, H. ; SHEN, F. ; GAO, F. ; HAN, W.** Learning efficient, explainable and discriminative representations for pulmonary nodules classification. *Pattern Recognition,* 2021, vol. 113, 107825
- **SHAMSHAD, F. ; KHAN, S. ; ZAMIR, S.W. ; KHAN, M.H. ; HAYAT, M ; KHAN, F.S. ; FU, H.** Transformers in medical imaging: A survey. *arXiv preprint arXiv:2201.09873,* 2022
- **TONEKABONI, S. ; JOSHI, S. ; MCCRADDEN, M.D. ; GOLDENBERG, A.** What clinicians want: contextualizing explainable machine learning for clinical end use. *Machine learning for healthcare conference,* 2019, 359-380

- **ZHENG, S. ; SHEN, Z. ; PEI, C. ; DING, W. ; LIN, H. ; ZHENG, J. ; PAN, L. ; ZHENG, B. ; HUANG, L.** Interpretative computer-aided lung cancer diagnosis: From radiology analysis to malignancy evaluation. *Computer Methods and Programs in Biomedicine,* 2021, vol. 210, 106363
- Generation of multimodal justification using visual word constraint model for explainable computer-aided diagnosis. **LEE, H. ; KIM, S.T. ; RO, Y.M.** Interpretability of Machine Intelligence in Medical Image Computing and Multimodal Learning for Clinical Decision Support. Springer, 2019, 21-29
- **WANG, X. ; PENG, Y. ; LU, L. ; LU, Z. ; SUMMERS, R.M.** Tienet: Text-image embedding network for common thorax disease classification and reporting in chest x-rays. *Proceedings of the IEEE conference on computer vision and pattern recognition,* 2018, 9049-9058
- **SHAMSHAD, F. ; KHAN, S ; ZAMIR, S.W. ; KHAN, M.H. ; HAYAT, M. ; KHAN, F.S. ; FU, H.** Transformers in medical imaging: A survey. *arXiv preprint arXiv:2201.09873,* 2022
- **MARQUES, S. ; SCHIAVO, F. ; FERREIRA, C.A. ; PEDROSA, J. ; CUNHA, A. ; CAMPILHO, A.** A multi-task CNN approach for lung nodule malignancy classification and characterization. *Expert Systems with Applications,* 2021, vol. 184, 115469
- **J. WARING ; C. LINDVALL ; R. UMETON.** Automated machine learning: Review of the state-of-the-art and opportunities for healthcare. *Artificial intelligence in medicine,* 2020, vol. 104, 101822
- **M. CLAESEN ; B. DE MOOR.** Hyperparameter search in machine learning. *arXiv preprint arXiv:1502.02127,* 2015
- **T. AKIBA ; S. SANO ; T. YANASE et al.** Optuna: A next-generation hyperparameter optimization framework. *Proceedings of the International Conference on Knowledge Discovery and Data Mining,* 2019
- **R. ANDONIE.** Hyperparameter optimization in learning systems. *Journal of Membrane Computing,* 2019, vol. 1 (4), 279-291
- **J. BERGSTRA ; Y. BENGIO.** Random search for hyper-parameter optimization. *Journal of machine learning research,* 2012, vol. 13 (2
- **J. WU ; X.-Y. CHEN ; H. ZHANG ; L.-D. XIONG ; H. LEI ; S.-H. DENG.** Hyperparameter optimization for machine learning models based on bayesian optimization. *Journal of Electronic Science and Technology,* 2019, vol. 17 (1), 26-40
- **N. M. ASZEMI ; P. DOMINIC.** Hyperparameter optimization in convolutional neural network using genetic algorithms. *International Journal of Advanced Computer Science and Applications,* 2019, vol. 10 (6
- Hyperparameter optimization: comparing genetic algorithm against grid search and bayesian optimization. **H. ALIBRAHIM ; S. A. LUDWIG.** 2021 IEEE Congress on Evolutionary Computation (CEC). IEEE, 2021, 1551-1559
- **C.-W. HSU ; C.-C. CHANG ; C.-J. LIN et al.** *A practical guide to support vector classification,* 2003
- **D. CHICCO.** Ten quick tips for machine learning in computational biology. *BioData mining,* 2017, vol. 10 (1), 1-17
- **J. WAINER ; G. CAWLEY.** Nested cross-validation when selecting classifiers is overzealous for most practical applications. *Expert Systems with Applications,* 2021, vol. 182, 115222